(19) Europäisches Patentamt
European Patent Office
Office européen des brevets

(11) **EP 2 416 699 B1**

(12) **EUROPÄISCHE PATENTSCHRIFT**

(45) Veröffentlichungstag und Bekanntmachung des Hinweises auf die Patenterteilung:
**26.03.2014 Patentblatt 2014/13**

(21) Anmeldenummer: **10722927.0**

(22) Anmeldetag: **08.04.2010**

(51) Int Cl.:
***A61B 5/00*** (2006.01)    ***A61M 1/16*** (2006.01)

(86) Internationale Anmeldenummer:
**PCT/EP2010/002188**

(87) Internationale Veröffentlichungsnummer:
**WO 2010/115621 (14.10.2010 Gazette 2010/41)**

(54) **VORRICHTUNG UND VERFAHREN ZUR MESSUNG EINES BLUTBESTANDTEILS IM BLUT FÜR EINE EXTRAKORPORALE BLUTBEHANDLUNGSVORRICHTUNG**

DEVICE AND METHOD FOR MEASURING A BLOOD CONSTITUENT IN BLOOD FOR AN EXTRACORPOREAL BLOOD TREATMENT DEVICE

DISPOSITIF ET PROCÉDÉ DE MESURE D'UN COMPOSANT SANGUIN DANS LE SANG POUR UN DISPOSITIF EXTRACORPOREL DE TRAITEMENT DU SANG

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **11.04.2009 DE 102009017304**

(43) Veröffentlichungstag der Anmeldung:
**15.02.2012 Patentblatt 2012/07**

(73) Patentinhaber: **Fresenius Medical Care Deutschland GmbH**
**61352 Bad Homburg v.d.H. (DE)**

(72) Erfinder: **ZHANG, Wei**
**97464 Niederwerrn (DE)**

(74) Vertreter: **Oppermann, Frank et al**
**OANDO Oppermann & Oppermann LLP**
**Washingtonstrasse 75**
**65189 Wiesbaden (DE)**

(56) Entgegenhaltungen:
WO-A1-2004/057313    US-A- 5 312 550
US-A1- 2004 129 616    US-A1- 2007 083 145
US-B1- 6 222 189    US-B1- 6 587 704

**Beschreibung**

Die Erfindung betrifft eine Vorrichtung zur Messung eines Blutbestandteils im Blut für eine extrakorporale Blutbehandlungsvorrichtung, die einen durch eine semipermeable Membran in eine erste Kammer und eine zweite Kammer unterteilten Dialysator oder Filter und ein Schlauchleitungssystem umfasst, das für elektromagnetische Strahlung durchlässige Schlauchleitungen aufweist.

Zur Entfernung von harnpflichtigen Substanzen und zum Flüssigkeitsentzug werden beim chronischen Nierenversagen verschiedene Verfahren zur extrakorporalen Blutbehandlung bzw. -reinigung eingesetzt. Bei der Hämodialyse wird das Blut des Patienten außerhalb des Körpers in einem Dialysator gereinigt. Der Dialysator weist eine Blutkammer und eine Dialysierflüssigkeitskammer auf, die von einer semipermeablen Membran getrennt sind. Während der Behandlung strömt das Blut des Patienten durch die Blutkammer. Um das Blut effektiv von den harnpflichtigen Substanzen zu reinigen, wird die Dialysierflüssigkeitskammer kontinuierlich von frischer Dialysierflüssigkeit durchströmt.

Während bei der Hämodialyse (HD) der Transport der kleinmolekularen Substanzen durch die Membran des Dialysators im Wesentlichen durch die Konzentrationsunterschiede (Diffusion) zwischen der Dialysierflüssigkeit und dem Blut bestimmt wird, werden bei der Hämofiltration (HF) im Plasmawasser gelöste Substanzen, insbesondere höhermolekulare Stoffe durch einen hohen Flüssigkeitsstrom (Konvektion) durch die Membran des Dialysators effektiv entfernt. Bei der Hämofiltration fungiert der Dialysator als Filter. Eine Kombination aus beiden Verfahren ist die Hämodiafiltration (HDF).

Bei Dialysepatienten treten zusätzlich zum Nierenversagen häufig Begleiterkrankungen auf, zu denen in einem Drittel aller Fälle die Diabetes-Mellitus zählt. Um weitere Folgeschäden zu minimieren, ist eine optimale Einstellung der Diabetes-Therapie erforderlich. Die Diagnose einer Diabetes-Mellitus und Überwachung der Therapie erfolgt mittels der Messung des Blutzuckers (Blutglukose).

Zur Messung der Blutglukose sind sowohl invasive als auch nicht-invasive Verfahren bekannt. Bekannte nicht-invasive Methoden zur Bestimmung der Blutglukose beruhen auf der Messung der Transmission von Licht im Blut des Patienten. Im infraroten Bereich liegen die Glukose-Absorptionsbände bei 760 nm, 920 nm und 1000 nm. Die Absorptionen sind jedoch so schwach, dass sie kaum nachweisbar sind. Daher wird von einer sogenannten künstlichen Blutkinetik Gebrauch gemacht.

Zur Messung der Konzentration von Glukose im Blut des Patienten findet bei den bekannten nicht-invasiven Verfahren eine Messanordnung Verwendung, die eine an den Finger des Patienten anzulegende Druckmanschette mit einer Lichtquelle und optischen Sensoren für eine Transmissionsmessung aufweist. Die Druckmanschette am Finger des Patienten wird kurzzeitig mit einem Druck beaufschlagt, der über dem systolischen Druck liegt, so dass der Blutfluss im Finger gestoppt wird, wodurch eine sogenannte künstliche Blutkinetik erzeugt wird. Die Erythrozyten sammeln sich in Gruppen an, so dass die effektive Größe der Streukörper zunimmt. Dadurch ist es möglich, auf der Grundlage einer Transmissionsmessung die Konzentration von Glukose im Blut zu bestimmen.

Die bekannten Messverfahren sehen unterschiedliche Auswertungen der einzelnen Messergebnisse vor. Allen Messverfahren ist aber gemeinsam, dass eine Transmissionsmessung am Finger des Patienten erfolgt, während der Finger des Patienten mit einer Druckmanschette zur Erzeugung einer künstlichen Blutkinetik mit Druck beaufschlagt wird.

Die oben beschriebenen Verfahren zur Messung der Konzentration von Glukose im Blut sind beispielsweise im Einzelnen beschrieben in dem Artikel: "Ilya Fine, et al: Occlusion Spectroscopy as a New Paradigm for Non-Invasive Blood Measurement, Proceedings of SPIE, Vol. 4263, pp. 122 - 130, 2001". Im Einzelnen sind die bekannte Messverfahren zur Messung der Glukosekonzentration auch in der WO 2006/006153 A1, WO 2007/020647 A1 und WO 2004/105596 Albeschrieben.

Die WO 2004/105596 A1 beschreibt ein Verfahren zur Messung der Glukosekonzentration, bei dem der Blutfluss im Finger durch eine erste Druckmanschette gestoppt wird und durch eine zweite Druckmanschette, die zwischen der ersten Manschette und der Fingerspitze angeordnet ist, der Blutfluss in der Fingerspitze moduliert wird. Dadurch wird in der Fingerspitze eine künstliche Blutkinetik erzeugt, die sich auf die Transmissionsmessung auswirkt und zur Berechnung des Hämoglobinwertes herangezogen wird.

Ein Verfahren zur Bestimmung der Konzentration von Glukose in einer Dialysierflüssigkeit während einer Dialysebehandlung ist aus der EP 1 083 948 B1 bekannt. Das bekannte Verfahren setzt aber die Entnahme einer Dialysierflüssigkeitsprobe während der Dialysebehandlung voraus.

Die bekannten Verfahren zur Glukosemessung haben sich in der Praxis bewährt. Nachteilig ist jedoch, dass eine Druckmanschette am Finger des Patienten angebracht oder eine Probe entnommen werden muss.

Aus der US 2004/129616 A1 und der US 2007/083145 A1 ist eine Dialysevorrichtung mit einem extrakorporalen Blutkreislauf bekannt, wobei stromauf und stromab der Dialysierflüssigkeitskammer an der Blutzuführ- bzw. Blutrückführleitung optische Messeinrichtungen zum Messen des Hämatokrit vorgesehen sind. Die Hämodialysemaschine sieht auch eine Veränderung der Blutflussrate und der Ultrafiltrationsrate durch die Einstellung unterschiedlicher Drehzahlen für die Blut- und Ultrafiltrationspumpe vor.

Die US 5,312,550 beschreibt eine Vorrichtung zur Bestimmung der Konzentration eines Blutbestandteils, die auf der Injektion einer Lösung zur Veränderung der Eigenschaften des Bluts beruht.

Der Erfindung liegt die Aufgabe zugrunde, eine Vorrichtung zu schaffen, mit der eine nicht-invasive Messung eines Blutbestandteils, beispielsweise der Konzentration von Glukose im Blut, während einer extrakorporalen Blutbehandlung mit einer extrakorporalen Blutbehandlungsvorrichtung möglich ist.

Die Lösung dieser Aufgabe erfolgt erfindungsgemäß mit den Merkmalen des Patentanspruchs 1. Vorteilhafte Ausführungsformen sind Gegenstand der Unteransprüche.

Die erfindungsgemäße Vorrichtung macht davon Gebrauch, dass die in den bekannten extrakorporalen Blutbehandlungsvorrichtungen verwendeten Schlauchleitungssysteme im allgemeinen Schlauchleitungen sind, die für elektromagnetische Strahlung, insbesondere Licht durchlässig sind. Die Erfindung beruht darauf, dass die Kinetik der an einer Messstelle in mindestens einer Schlauchleitung des Schlauchleitungssystems fließenden Flüssigkeit verändert wird. Dies kann durch Verändern des Strömungsverhaltens der Flüssigkeit in der mindestens einen Schlauchleitung des Schlauchleitungssystems an der Messstelle erfolgen. Die eigentliche Analyse der gewonnenen Messdaten zur Bestimmung der Konzentration des Blutbestandteils erfolgt dann nach den bekannten Verfahren, die von einer Manschette am Finger des Patienten Gebrauch machen, wobei die Erfindung allerdings die Intensität der in die Schlauchleitung an der Messstelle eintretenden und aus der Schlauchleitung an der Messstelle austretenden elektromagnetischen Strahlung für verschiedene Wellenlängen analysiert. Die Veränderung des Strömungsverhaltens der in der Schlauchleitung fließenden Flüssigkeit führt dazu, dass mit einer Transmissions-, Reflektions- und Streulichtmessung der Blutbestandteil bestimmt werden kann.

Die erfindungsgemäße Vorrichtung kann mit Ausnahme der Messanordnung von den Teilen Gebrauch machen, die bereits in den bekannten extrakorporalen Blutbehandlungsvorrichtungen vorhanden sind. Zu denen zählen beispielsweise die zentrale Steuer- und Recheneinheit, mit der die für die Messung erforderlichen Einstellungen vorgenommen werden können und die Analyse der gewonnenen Messdaten erfolgen kann. Der entscheidende Vorteil der erfindungsgemäßen Vorrichtung liegt darin, dass eine nicht-invasive Messung des Blutbestandteils vor oder nach oder während der extrakorporalen Blutbehandlung möglich ist, ohne jedoch eine Druckmanschette an den Finger des Patienten anlegen oder eine Dialysierflüssigkeitsprobe nehmen zu müssen.

Die extrakorporale Blutbehandlung macht einen kontinuierlichen Zugang zum Blut des Patienten möglich. Da verschiedene Blutbestandteile, beispielsweise Glukose, den Dialysator oder Filter einer extrakorporalen Blutbehandlungsvorrichtung passieren können, kann die Messung des Blutbestandteils grundsätzlich sowohl im extrakorporalen Blutkreislauf als auch im Dialysierflüssigkeitskreislauf der Blutbehandlungsvorrichtung erfolgen. Vorzugsweise wird die Messung aber in mindestens einer Schlauchleitung des Schlauchleitungssystems des extrakorporalen Blutkreislaufs, insbesondere in der zur Blutkammer des Dialysators oder Filters der Blutbehandlungsvorrichtung führenden Blutzuführleitung vorgenommen.

Die Erfindung sieht vor, das Strömungsverhalten des in der Blutzuführ- oder Blutrückführleitung fließenden Bluts dadurch zu verändern, dass die Förderrate der im extrakorporalen Blutkreislauf angeordneten Blutpumpe, die insbesondere in der Blutzuführleitung angeordnet ist, verändert wird. Vorzugsweise wird die Blutpumpe für ein kurzes Zeitintervall, beispielsweise für 2 bis 20 Sekunden, insbesondere 8- 12 Sekunden gestoppt. Die Blutpumpe braucht aber nicht vollständig gestoppt werden, sondern der Blutfluss nur abrupt reduziert werden. Es ist grundsätzlich auch möglich, die Förderrate der Blutpumpe kurzzeitig zu ändern, insbesondere kurzeitig zu erhöhen und zu verringern, um das Strömungsverhalten zu verändern. Beispielsweise kann der Blutfluss von beispielsweise 250 ml/min auf 400 ml/min erhöht und dann auf 100 ml/ml reduziert werden, bevor wieder der Blutfluss von 250 ml/min eingestellt wird.

Bei einer bevorzugten Ausführungsform wird sowohl die Blutpumpe für ein vorgegebenes kurzes Zeitintervall gestoppt als auch ein im extrakorporalen Blutkreislauf, insbesondere in der Blutzuführleitung stromauf der Blutpumpe angeordnetes Absperrorgan, beispielsweise eine Schlauchklemme geschlossen, wobei die Messung in dem Abschnitt der Schlauchleitung stromauf des Absperrorgans erfolgt. Anschließend wird das Absperrorgan wieder geöffnet und die Blutpumpe wieder in Betrieb gesetzt. Es ist auch möglich, zur künstlichen Veränderung der Blutkinetik an der Messstelle das Absperrorgan mehrfach zu öffnen und zu schließen. Vorzugsweise wird das Absperrorgan vollständig geschlossen, es ist aber auch möglich, das Absperrorgan nur teilweise zu schließen, so dass die Schlauchleitung nicht vollständig abgeklemmt ist. Allein entscheidend ist, dass die Kinetik des Bluts in der Schlauchleitung signifikant verändert wird, so dass mit den bekannten Messverfahren auf der Grundlage der Transmissions-, Reflektions- oder Streulichtmessung der Blutbestandteil bestimmt werden kann.

Im Folgenden wird die Erfindung unter Bezugnahme auf die Zeichnungen näher erläutert. Es zeigen:

Fig. 1    die wesentlichen Bestandteile einer extrakorporalen Blutbehandlungsvorrichtung zusammen mit der Vorrichtung zur Messung eines Blutbestandteils im Blut in stark vereinfachter schematischer Darstellung,

Fig. 2    die Messanordnung der Vorrichtung zur Messung eines Blutbestandteils in einer perspektivischen, stark vereinfachten schematischen Darstellung,

Fig. 3    eine schematische Darstellung der Messanordnung der Vorrichtung zur Messung eines Blutbestandteils in der Draufsicht,

Fig. 4    die Messanordnung in geschnittener Darstellung,

Fig. 5    den Signalverlauf des mit der Messanordnung gemessenen Signals beim Stopp der Blutpumpe,

Fig. 6    die Abhängigkeit einer bei der Messung ermittelten ersten Zwischengröße $Y_1$ von der Glukosekonzentration bei einer Transmissionsmessung,

Fig. 7    die Abhängigkeit einer bei der Messung ermittelten ersten Zwischengröße $Y_1$ von der Glukosekonzentration bei einer Reflektionsmessung,

Fig. 8    die Abhängigkeit einer bei der Messung ermittelten zweiten Zwischengröße $Y_2$ von der Glukosekonzentration bei einer Transmissionsmessung und

Fig. 9    die Abhängigkeit einer bei der Messung ermittelten dritten Zwischengröße $Y_3$ von der Glukosekonzentration bei einer Transmissionsmessung.

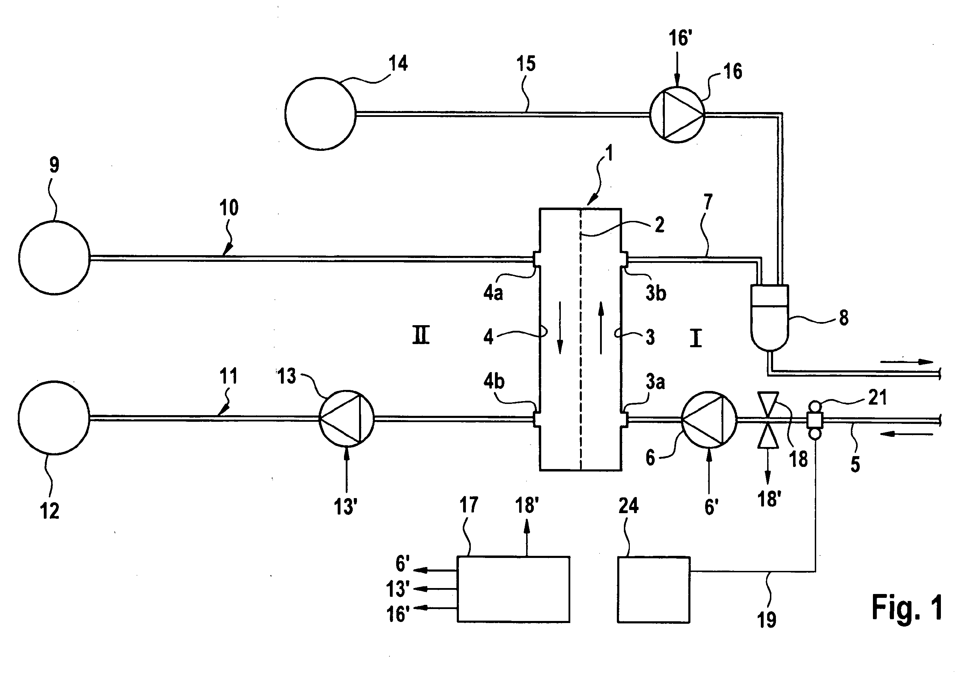

Fig. 1 zeigt in stark vereinfachter schematischer Darstellung die für die Erfindung relevanten Komponenten einer extrakorporalen Blutbehandlungsvorrichtung, die sowohl als Hämodialysevorrichtung und/oder Hämofiltrationsvorrichtung betrieben werden kann. Daher wird die extrakorporale Blutbehandlungsvorrichtung nachfolgend als Hämodiafiltrationsvorrichtung bezeichnet.

Die Hämodiafiltrationsvorrichtung weist einen Dialysator oder Filter 1 auf, der durch eine semipermeable Membran 2 in eine Blutkammer 3 und eine Dialysierflüssigkeitskammer 4 getrennt ist. Der Einlass 3a der Blutkammer ist mit einem Ende der arteriellen Blutzuführleitung 5 verbunden, in die eine Blutpumpe 6 geschaltet ist, während der Auslass 3b der Blutkammer mit einem Ende der venösen Blutrückführleitung 7 verbunden ist, in die eine Tropfkammer 8 geschaltet ist. An den anderen Enden der arteriellen und venösen Blutleitung 5, 7 befinden sich die nicht dargestellten arteriellen und venösen Kanülen zum Anschluss an den Patienten. Dieser Teil des Flüssigkeitssystems stellt den extrakorporalen Blutkreislauf I der Hämodiafiltrationsvorrichtung dar. Bei den Blutleitungen 5, 7 handelt es sich um für Licht im Wesentlichen durchlässige Schlauchleitungen aus einem ausreichend transparenten Material.

Das Dialysierflüssigkeitssystem II der Hämodiafiltrationsvorrichtung umfasst eine Einrichtung 9 zur Bereitstellung frischer Dialysierflüssigkeit, die über eine ebenfalls transparente Dialysierflüssigkeitszuführleitung 10 mit dem Einlass 4a der Dialysierflüssigkeitskammer 4 des Dialysators 1 oder Filters verbunden ist. Von dem Auslass 4b der Dialysierflüssigkeitskammer 4 des Dialysators 1 oder Filters geht eine transparente Dialysierflüssigkeitsrückführleitung 11 ab, die zu einem Auslass 12 führt. Zum Fördern der Dialysierflüssigkeit dient eine Dialysierflüssigkeitspumpe 13, die in der Dialysierflüssigkeitsrückführleitung 11 angeordnet ist.

Darüber hinaus verfügt die Hämodiafiltrationsvorrichtung über eine Substituatquelle 14, von der eine Substituatleitung 15, in die eine Substituatpumpe 16 geschaltet ist, zu der venösen Tropfkammer 8 führt. Mit der Substituatpumpe 16 kann dem extrakorporalen Blutkreislauf I eine vorgegebene Menge an Substitutionsflüssigkeit aus der Substituatquelle 14 zugeführt werden, wenn dem Blutkreislauf über den Dialysator 1 Flüssigkeit entzogen wird.

Die Diafiltrationsvorrichtung umfasst weiterhin eine zentrale Steuer- und Recheneinheit 17, die über Steuerleitungen 6', 13', 16' mit der Blutpumpe 6, der Dialysierflüssigkeitspumpe 13 und der Substituatpumpe 16 verbunden ist. Die Steuer- und Recheneinheit 17 sendet an die einzelnen Komponenten Steuerbefehle und empfängt von den Komponenten Daten über deren Betriebszustände, beispielsweise die Förderraten der Pumpen.

Nachfolgend wird die erfindungsgemäße Vorrichtung zur Messung eines Blutbestandteils im Blut beschrieben, die eine selbständige Einheit bilden oder Bestandteil der extrakorporalen Blutbehandlungsvorrichtung sein kann. Bei dem vorliegenden Ausführungsbeispiel ist die erfindungsgemäße Vorrichtung Bestandteil der extrakorporalen Blutbehandlungsvorrichtung. Hier dient die erfindungsgemäße Vorrichtung zur Messung der Konzentration von Glukose im Blut des Patienten, das über die arterielle Blutleitung 5 in die Blutkammer 3 des Dialysators 1 strömt. Es ist aber grundsätzlich auch möglich mit der erfindungsgemäßen Vorrichtung andere Blutbestandteile als Glukose zu messen.

Die Vorrichtung zur Messung von Glukose verfügt über eine in Fig. 1 nur andeutungsweise dargestellte Messanordnung 21, die in dem Abschnitt der arteriellen Blutleitung 5 stromauf der Blutpumpe 6 angeordnet ist. Zwischen der Blutpumpe 6 und der Messanordnung 21 befindet sich in der arteriellen Blutleitung 5 ein Absperrorgan 18, insbesondere eine elektromagnetisch betätigbare Schlauchklemme, mit der die Schlauchleitung teilweise oder vollständig abgeklemmt werden kann. Die elektromagnetisch betätigbare Schlauchklemme 18 ist über eine Steuerleitung 18' mit der zentralen Steuer- und Recheneinheit 17 verbunden. Folglich ist die Messanordnung 21 in der arteriellen Blutleitung 5 stromauf des Absperrorgans 18 angeordnet.

Die Vorrichtung zur Messung von Glukose verfügt weiterhin über eine Analyseeinheit 24, die über eine Datenleitung 19 mit der Messanordnung 21 verbunden ist. Die Analyseeinheit 24 analysiert die Messdaten der Messanordnung 21 und bestimmt die Konzentration von Glukose im Blut, die auf einer nicht dargestellten Anzeigeeinheit angezeigt wird.

Für die Erfindung ist es nicht wesentlich, wie die mit der Messanordnung gewonnenen Messdaten ausgewertet werden. Entscheidend ist aber, dass die Messung nach den bekannten Verfahren dadurch möglich ist, dass nicht in dem Finger des Patienten, sondern in der arteriellen Blutleitung 5 die Kinetik des Bluts künstlich verändert wird. Beispielsweise können die in der WO 2006/006153 A1 oder WO 2007/020647 A1 beschriebenen Verfahren zur Messung eines Blutbestandteils herangezogen werden.

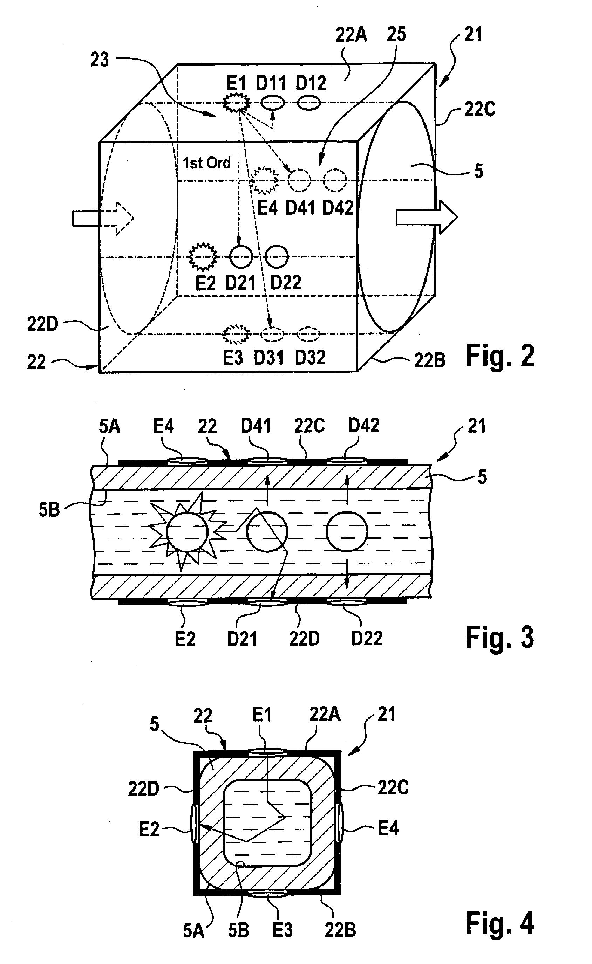

Die Fig. 2 bis 4 zeigen die Messanordnung 21 in vergrößerter schematischer Darstellung. Es handelt sich um eine Messanordnung, die in der WO 2004/057313 A1 im Einzelnen beschrieben ist.

Während der Messung ist die arterielle Blutleitung 5 in der Messanordnung 21 eingespannt, die mit Blut gefüllt ist. Die Messanordnung 21 verfügt hierfür über eine Einspannvorrichtung 22 mit vier senkrecht aufeinander stehenden, planaren Anlageflächen 22A, 22B, 22C, 22D, zwischen denen die Schlauchleitung 5 eingespannt werden kann. Die Einspannvorrichtung 22 ist derart bemessen, dass sich die Schlauchleitung 5 so verformen kann, dass sie eine vorzugsweise plane Außen- und Innenfläche 5A, 5B aufweist. Darüber hinaus verfügt die Messanordnung 21 über einen Sender 23 zum Senden von elektromagnetischer Strahlung, der insbesondere mehrere Lichtquellen E1, E2, E3, E4 umfasst, sowie einen Empfänger 25 für elektromagnetische Strahlung, der insbesondere mehrere Lichtdetektoren D11, D21, D31, D41, D12, D22, D32, D42 umfasst. Die Lichtquellen bilden zusammen mit den Lichtdetektoren eine Messvorrichtung für eine Transmissionsmessung, eine Messvorrichtung für eine Streulichtmessung und eine Messvorrichtung für eine Reflektionsmessung.

Die Einspannvorrichtung 22 weist an der Oberseite und der Unterseite sowie an den Längsseiten jeweils eine Reihe von drei in gleichbleibenden Abständen zueinander angeordneten Bohrungen auf, in denen die Lichtquellen und Lichtdetektoren jeweils angeordnet sind.

Die Lichtquellen E1, E2, E3, E4, insbesondere LEDs, sind nach Figur 2 in der in Flussrichtung jeweils ersten Bohrung angeordnet, während die Lichtdetektoren D11, D12, D21, D22, D31, D32, D41, D42, insbesondere Photodioden, in den in Flussrichtung jeweils zweiten und dritten Bohrungen angeordnet sind. Ebenso ist es möglich, die Position der Lichtquellen und der Lichtdetektoren relativ zur Flussrichtung zu vertauschen.

Die LEDs E1, E2, E3, E4 senden Licht mit zwei unterschiedlichen Wellenlängen bevorzugt $\lambda_1$ = 610 nm /670 nm und $\lambda_2$ = 805 nm aus, das von den Photodioden D11, D12, D21, D22, D31, D32, D41, D42 als durch die blutgefüllte Schlauchleitung hindurchtretendes Licht (Transmissionsmessung), als in der blutgefüllten Schlauchleitung gestreutes Licht (Streulichtmessung) und als in der blutgefüllten Schlauchleitung reflektiertes Licht (Reflektionslichtmessung) erfasst wird.

Für die Messung der Glukose wird in der blutgefüllten Schlauchleitung an der Messstelle eine künstliche Blutkinetik erzeugt. Bei einer bevorzugten Ausführungsform der Erfindung steuert die zentrale Steuer- und Recheneinheit 17 die Blutpumpe 6 derart an, dass die Blutpumpe für ein kurzes Zeitintervall, insbesondere für 10 Sekunden angehalten wird. Anschließend wird die Blutpumpe wieder in Betrieb gesetzt. Dadurch wird die Blutkinetik maximiert, wodurch sich ein verbessertes Signal-Rausch-Verhältnis ergibt. Die roten Blutkörperchen orientieren sich nach Verschwinden der Schwerkraft durch den Stopp der Blutpumpe neu und sedimentieren überwiegend.

Eine alternative Ausführungsform sieht zur Veränderung der Blutkinetik vor, dass die zentrale Steuer- und Recheneinheit 17 die Blutpumpe 6 derart ansteuert, dass die Förderate der Blutpumpe für ein kurzes erstes Zeitintervall, beispielsweise von 250 ml/min auf 400 ml/min erhöht wird und anschließend für ein kurzes zweites Zeitintervall, beispielsweise auf 100 ml/min verringert wird, wobei dann wieder die ursprüngliche Förderrate eingestellt wird.

Eine weitere alternative Ausführungsform sieht anstelle eines vollständigen Stopps der Blutpumpe 6 nur eine drastische Reduzierung der Förderrate der Blutpumpe vor. Beispielsweise wird die Förderrate der Blutpumpe von 250 ml/min auf mindestens 100 ml/ min reduziert werden. Allerdings ergibt sich bei dieser Ausführungsform ein schlechteres Signal-Rausch-Verhältnis als bei einem vollständigen Pumpenstopp.

Bei der Ausführungsform, die Gegenstand der Erfindung ist, steuert die zentrale Steuer- und Recheneinheit 17 die Blutpumpe 6 und die elektromagnetisch betätigbare Schlauchklemme 18 derart an, dass für ein vorgegebenes kurzes Zeitintervall die Blutpumpe 6 gestoppt und die Schlauchklemme 18 dann in dem vorgegebenen kurzen Zeitintervall vorzugsweise vollständig oder zumindest teilweise geschlossen wird. Dadurch werden die Strömungsverhältnisse an der Messstelle in dem Abschnitt der arteriellen Blutleitung 5 stromauf der Schlauchklemme 18 verändert. Daraufhin wird die Blutpumpe 6 wieder in Gang gesetzt und die Schlauchklemme 18 wird wieder geöffnet. Das Schließen bzw. Öffnen der Schlauchklemme kann während der Messung bei angehaltener Blutpumpe 6 fortlaufend erfolgen, d.h. nach dem Anhalten der Blutpumpe wird die Schlauchklemme zum Zeitpunkt $t_{1n}$ geschlossen und zum Zeitpunkt $t_{2n}$ wird die Klemme geöffnet usw.

Zunächst wird das Messverfahren in allgemeiner Form beschrieben. Die Messanordnung führt bei den Wellenlängen $\lambda_1$ und $\lambda_2$ die nachfolgend bezeichneten Messungen durch, während die Blutkinetik nach einem der oben beschriebenen Verfahren künstlich verändert wird

Die Messanordnung 21 misst sowohl die Vorwärtsstreuung / Transmission, die Rückwärtsstreuung / Reflektion als auch die 90°-Seitwärtsstreuung. Alle Messungen erfolgen bei den Wellenlängen $\lambda_1$ und $\lambda_2$.

$FS_{\lambda1}(t)$ - Vorwärtsstreuung / Transmission bei Wellenlänge $\lambda_1$
$SS_{\lambda1}(t)$ - 90°-Seitwärtsstreuung bei Wellenlänge $\lambda_1$
$FS_{\lambda2}(t)$ - Vorwärtsstreuung / Transmission bei Wellenlänge $\lambda_2$
$SS_{\lambda2}(t)$ - 90°-Seitwärtsstreuung bei Wellenlänge $\lambda_2$
$BS_{\lambda1}(t)$ - Rückwärtsstreuung / Reflektion bei Wellenlänge $\lambda_1$
$BS_{\lambda2}(t)$ - Rückwärtsstreuung / Reflektion bei Wellenlänge $\lambda_2$

wobei $t \in (t_1, t_2)$

Aus den gewonnenen Messdaten für die Vorwärts-, Rückwärts- und Seitwärtsstreuung berechnet die Analyseeinheit 24 zumindest eine der nachfolgenden Zwischengrößen:

$$x = S_{\lambda1}(t) / S_{\lambda2}(t), S = FS, BS, SS$$

$$y = \frac{dS_{\lambda1}(t)}{dt} \Big/ \frac{dS_{\lambda2}(t)}{dt}, S = FS, BS, SS$$

$$\rightarrow z = \frac{FS_{\lambda1}(t) \big/ S_{\lambda1}(t)}{FS_{\lambda2}(t) \big/ S_{\lambda2}(t)}, S = BS, SS$$

Aus den berechneten Zwischengrößen wird die Konzentration von Glukose im Blut des Patienten dann nach den bekannten Verfahren ermittelt:

$$C_{glucose}(t) = g_1(x)$$

oder

$$C_{glucose}(t) = g_2(y)$$

oder

$$C_{glucose}(t) = g_3(z)$$

Im Gegensatz zu Patienten, deren Hämoglobin bei der Glukosemessung nahezu konstant bleibt, kann sich das Hämoglobin bei Dialysepatienten im Verlauf der Dialysebehandlung wegen der Ultrafiltration ändern. In der Praxis zeigen sich Änderungen des Hämoglobins bis zu 20 %. Diese Änderungen des Hämoglobins haben einen relativ großen Einfluss auf die Genauigkeit der Glukosemessung. Daher sieht die erfindungsgemäße Vorrichtung zur Glukosemessung eine entsprechende Kompensation vor.

Während der Dialysebehandlung wird vorzugsweise kontinuierlich das Hämoglobin gemessen. Die Messung des Hämoglobins kann mit der gleichen Messanordnung 21 wie die Glukosemessung erfolgen. Die Messung des Hämoglobins $C_{HB}(t)$ nach den bekannten Verfahren erfolgt aber auf der Grundlage der Messung der 90°-Seitwärtsstreuung bei einer bestimmten Wellenlänge, wobei die Blutkinetik nicht verändert wird.

$$C_{HB}(t) = f(SS(t)) \qquad t \in (t_1, t_2).$$

Nachdem das Hämoglobin $C_{HB}(t)$ bestimmt ist, wird der nach dem oben beschriebenen Verfahren ermittelte Glukosewert in Abhängigkeit von dem Hämoglobin kompensiert. Hierzu sind entsprechende Korrekturfaktoren vorgesehen, die empirisch ermittelt worden sind und in einem Speicher der Analyseeinheit 24 gespeichert sind.

Nachfolgend wird ein Ausführungsbeispiel der erfindungsgemäßen Vorrichtung und das Verfahren, nach dem die Vorrichtung arbeitet, im Einzelnen beschrieben.

Die Messungen wurden mit der oben beschriebenen Messanordnung 21, die aus der WO 2004/057313 A1 bekannt ist, in Laborversuchen mit Rinderblut vorgenommen, das auf 37° C temperiert wurde. Die künstliche Veränderung der Blutkinetik an der Messstelle wurde durch einen kurzzeitigen Stopp der Blutpumpe 6 erzeugt.

Mit der Messanordnung 21 wurden die nachfolgend genannten Messungen durchgeführt, wobei mit der Analyseeinheit 24 die nachfolgenden Zwischengrößen $y_1(t)$, $y_2(t)$, $y_3(t)$ berechnet wurden. Dabei wurde eine Messung der Glukose mit nur einer einzigen Wellenlänge oder mit zwei Wellenlängen vorgenommen.

Bei der Einzelwellenlängenmessung wird die Zwischenvariable zur Bildung der Korrelation mit Glukose wie folgt definiert:

$$y_1(t_1) = s(t_1) - s(t_2) \tag{1}$$

mit $t_1$ - kurz vor einem Blutpumpenstopp,
$t_2$ - kurz nach dem Blutpumpenstopp,
s - kann der Signaltyp von Transmission/Vorwärtsstreuung (FS), Seitwärtsstreuung (SS) und Reflektion/Rückwärtsstreuung (BS) sein.

Der Zusammenhang zwischen der Variable und der Blutglukosekonzentration, der experimentell ermittelt werden kann, ergibt sich wie folgt:

$$C_{glucose}(t_1) = f_1(y_1(t_1))$$

Bei der Zweiwellenlängenmessung wird die Zwischenvariable zur Bildung der Korrelation mit Glukose wie folgt definiert:

$$y_2(t_1) = \frac{s_{\lambda 1}(t_1) - s_{\lambda 1}(t_2)}{s_{\lambda 2}(t_1) - s_{\lambda 2}(t_2)} \tag{2}$$

oder

$$y_3(t_1) = \frac{s_{\lambda 1}(t_1)}{s_{\lambda 2}(t_1)} - \frac{s_{\lambda 1}(t_2)}{s_{\lambda 2}(t_2)} \tag{3}$$

Der Zusammenhang zwischen der Variable und Blutglukosekonzentration, der experimentell ermittelt werden kann, ergibt sich wie folgt

$$C_{glu\cos e}(t_1) = f_2(y_2(t_1))$$

oder

$$C_{glu\cos e}(t_1) = f_3(y_3(t_1))$$

Die Messung kann beispielsweise mit den unterschiedlichen Wellenlängen $\lambda_1$ = 610 nm / 670 nm und $\lambda_2$ = 805 nm erfolgen.

Der Glukosegehalt kann aus den ermittelten Zwischengrößen anhand einer empirisch vorgenommenen Zuordnung ermittelt werden. Zum Abgleich der Analyseeinheit 24 und der Messanordnung 21 wird die Glukosekonzentration von Humanspenderblut künstlich definiert verändert. Die ermittelten Zwischengrößen werden dann dem bekannten Glukosegehalt zugeordnet. Die gewonnene Abbildung der Zwischenwerte auf den Glukosegehalt kann als eine Funktion in einem Speicher der Analyseeinheit 24 hinterlegt werden, um später nach jeder Messung den Glukosegehalt berechnen zu können. Hierzu ist im Allgemeinen eine lineare Gleichung ausreichend. Es ist aber auch möglich, die Zuordnung in Form einer Tabelle (look-up table) zu hinterlegen, in der Zwischengröße und Bestimmungsgröße aufeinander abgebildet werden.

Die Kalibrierung der erfindungsgemäßen Vorrichtung braucht nicht individuell für jede Vorrichtung vorgenommen zu werden. In der Praxis ist ausreichend, wenn die Zuordnung von Zwischengröße und Glukosegehalt in einer Referenzanordnung ermittelt wird. Um jedoch die individuellen Herstellungstoleranzen auszugleichen, beispielsweise unterschiedliche Abstände der LEDs und Photodioden der Messanordnung 21, kann jede Vorrichtung zur Glukosemessung auch individuell werksseitig kalibriert werden, indem ein Referenzmuster mit definierten Eigenschaften gemessen wird. Hierzu kann Humanblut, aber auch eine Ersatzflüssigkeit, insbesondere Rinderblut benutzt werden.

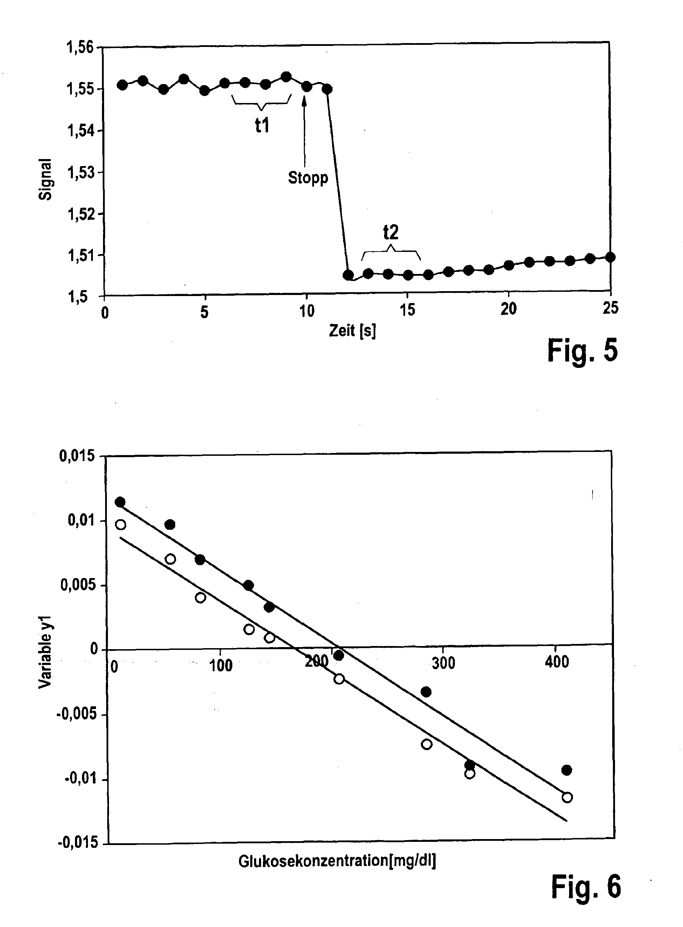

Fig. 5 zeigt den mit der Messanordnung 21 bei einer Transmissionsmessung gemessenen Signalverlauf, wenn die Blutpumpe 6 gestoppt wird. Es zeigt sich, dass sich das Signal nach dem Stopp der Blutpumpe sprunghaft verringert. Zur Ermittlung der Glukosekonzentration wird der Betrag des Signals kurz vor dem Stopp der Blutpumpe, beispielsweise in dem Zeitintervall $t_1$, und unmittelbar nach dem Stopp der Pumpe, beispielsweise in dem Zeitintervall $t_2$, von der Analyseeinheit 24 zur Bestimmung der Zwischenvariable ausgewertet.

Fig. 6 zeigt das Ergebnis der Messung für den Fall einer Transmissionsmessung mit der Messanordnung 21 bei nur einer Wellenlänge $\lambda$ = 670 nm und einer Wellenlänge $\lambda$ = 805 nm (Einzelwellenlängenmessung). Das Ergebnis der Transmissionsmessung mit der Wellenlänge $\lambda$ = 670 nm ist mit einem Punkt und mit der Wellenlänge $\lambda$ = 805 nm mit einem Kreis gekennzeichnet. Die Zwischenvariable wurde nach Gleichung (1) aus dem Betrag des Signals kurz vor dem Stopp der Blutpumpe in dem Zeitintervall $t_1$, und unmittelbar nach dem Stopp der Pumpe in dem Zeitintervall $t_2$ berechnet. Dabei wurde die Blutpumpe bei einem Blutfluss von 300 ml/min angehalten. Der Korrelationskoeffizient beträgt 0,9735 bei einer Wellenlänge $\lambda$ = 670 nm und 0,9805 bei $\lambda$ = 805 nm.

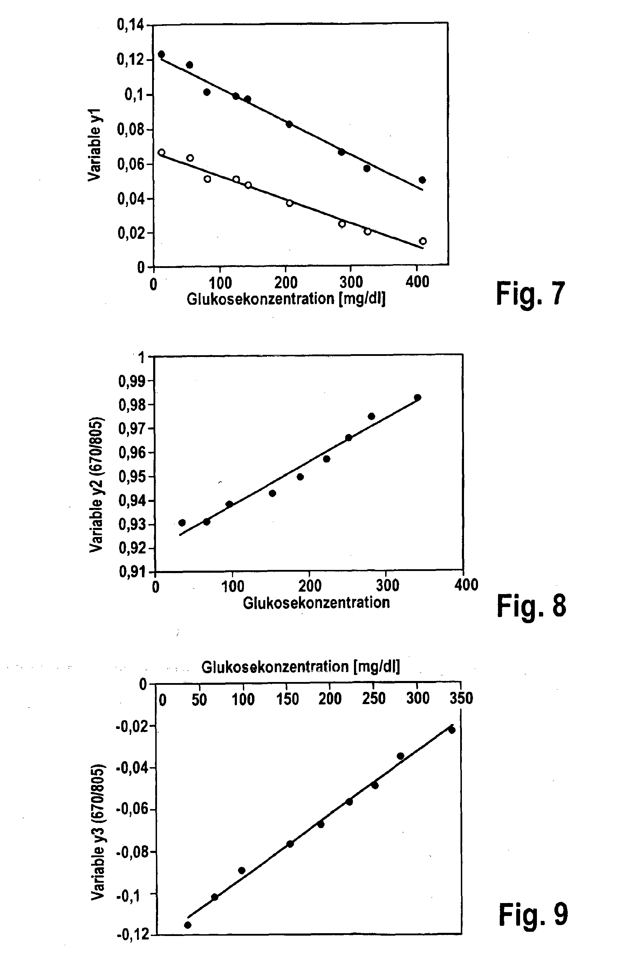

Fig. 7 zeigt das Ergebnis der Messung für den Fall einer Reflektionsmessung mit nur einer Wellenlänge $\lambda$ = 670 nm und einer Wellenlänge $\lambda$ = 805 nm (Einzelwellenlängenmessung). Das Ergebnis der Reflektionsmessung mit der Wellenlänge $\lambda$ = 670 nm ist mit einem Punkt und mit der Wellenlänge $\lambda$ = 805 nm mit einem Kreis gekennzeichnet. Die Zwischenvariable wurde nach Gleichung (1) aus dem Betrag des Signals kurz vor dem Stopp der Blutpumpe in dem Zeitintervall $t_1$, und unmittelbar nach dem Stopp der Pumpe in dem Zeitintervall $t_2$ berechnet. Dabei wurde die Blutpumpe bei einem Blutfluss von 300 ml/min angehalten. Der Korrelationskoeffizient beträgt 0,9771 bei einer Wellenlänge $\lambda$ = 670 nm und 0,9735 bei $\lambda$ = 805 nm.

Fig. 8 zeigt das Ergebnis der Messung für den Fall zweier Transmissionsmessungen mit zwei Wellenlängen $\lambda_1$ = 670 nm und $\lambda_2$ = 805 nm (Zweiwellenlängenmessung). Die Zwischenvariable wurde nach Gleichung (2) aus dem Betrag des Signals kurz vor dem Stopp der Blutpumpe in dem Zeitintervall $t_1$, und unmittelbar nach dem Stopp der Pumpe in dem Zeitintervall $t_2$ für die erste und zweite Wellenlänge $\lambda_1$ = 670 nm bzw. $\lambda_2$ = 805 nm berechnet. Dabei wurde die Blutpumpe bei einem Blutfluss von 200 ml/min angehalten. Der Korrelationskoeffizient beträgt 0,9713.

Fig. 9 zeigt das Ergebnis der Messung für den Fall zweier Transmissionsmessungen mit zwei Wellenlängen $\lambda_1$ = 670 nm und $\lambda_2$ = 805 nm (Zweiwellenlängenmessung). Die Zwischenvariable wurde nunmehr nach Gleichung (3) aus dem Betrag des Signals kurz vor dem Stopp der Blutpumpe in dem Zeitintervall $t_1$, und unmittelbar nach dem Stopp der Pumpe in dem Zeitintervall $t_2$ für die erste und zweite Wellenlänge $\lambda_1$ = 670 nm bzw. $\lambda_2$ = 805 nm berechnet. Dabei wurde die Blutpumpe wieder bei einem Blutfluss von 200 ml/min angehalten. Der Korrelationskoeffizient beträgt 0,9927.

Es zeigt sich, dass die Bestimmung des Glukosegehalts sowohl mit der Einzelwellenlängenmessung als auch der Zweiwellenlängenmessung vorgenommen werden kann, wobei mit der Messanordnung die Transmission, Reflektion und/oder Seitwärtsstreuung gemessen werden kann. Es ist ersichtlich, dass die Korrelation zwischen der Zwischenvariablen und der Bestimmungsgröße (Glukosekonzentration) bei der Anwendung der Gleichung (3) am besten ist. Unter Berücksichtigung des Einflusses der Hämoglobinkonzentration oder Sauerstoffsättigung wird die Messung mit zwei unterschiedlichen Wellenlängen bevorzugt.

**Patentansprüche**

1. Vorrichtung zur Messung eines Blutbestandteils im Blut für eine extrakorporale Blutbehandlungsvorrichtung, die einen durch eine semipermeable Membran in eine erste Kammer und eine zweite Kammer unterteilten Dialysator oder Filter und ein Schlauchleitungssystem umfasst, das eine zu der ersten Kammer (3) des Dialysators (1) oder Filters führende Blutzuführleitung (5) und eine von der ersten Kammer des Dialysators oder Filters abgehende Blutrückführleitung (7) aufweist, und das für elektromagnetische Strahlung im Wesentlichen durchlässige Schlauchleitungen aufweist,

   wobei die Vorrichtung zur Messung eines Blutbestandteils aufweist:

   eine Messanordnung (21) mit Mitteln (24) zum Senden von elektromagnetischer Strahlung, die in eine der Schlauchleitungen des Schlauchleitungssystems an einer Messstelle eintritt, und Mitteln (25) zum Empfangen von elektromagnetischer Strahlung, die an der Messstelle aus der Schlauchleitung austritt, wobei die Messanordnung für die Intensität der in die Schlauchleitung an der Messstelle eintretenden und aus der Schlauchleitung an der Messstelle austretenden elektromagnetischen Strahlung charakteristische Messdaten liefert,
   Mittel (6, 18) zum Verändern des Strömungsverhaltens der an der Messstelle in der Schlauchleitung fließenden Flüssigkeit,
   Mittel (24) zum Analysieren der während der Veränderung des Strömungsverhaltens gewonnenen Messdaten der Messanordnung (21) und Bestimmen der Konzentration des Blutbestandteils aus den Messdaten,
   **dadurch gekennzeichnet,**
   **dass** die Mittel (6, 18) zum Verändern des Strömungsverhaltens eine in der Blutzuführleitung (5) oder Blutrückführleitung (7) angeordnete Blutpumpe (6) zum Fördern von Blut, ein in der Blutzuführleitung (5) oder Blutrückführleitung (7) angeordnetes Absperrorgan (18) und Mittel (17) zum Ansteuern der Blutpumpe und des Absperrorgans aufweisen, die derart ausgebildet sind, dass die Flussrate des Bluts in der Blutzuführleitung bzw. Blutrückführleitung verändert wird, wobei die Messanordnung (21) an der Blutzuführleitung bzw. Blutrückführleitung angeordnet ist, und
   **dass** die Mittel (17) zum Ansteuern der Blutpumpe (6) und des Absperrorgans (18) derart ausgebildet sind, dass die Blutpumpe (6) für ein vorgegebenes Zeitintervall angehalten und nach dem Anhalten der Blutpumpe das Absperrorgan (18) zumindest teilweise, insbesondere vollständig, geschlossen und dann wieder geöffnet wird.

2. Vorrichtung nach Anspruch 1, **dadurch gekennzeichnet, dass** die Mittel (6, 18) zum Verändern des Strömungsverhaltens des in der Blutzuführleitung oder Blutrückführleitung fließenden Bluts eine in der Blutzuführleitung (5) angeordnete Blutpumpe (6) zum Fördern von Blut und ein in der Blutzuführleitung (5) angeordnetes Absperrorgan (18) aufweisen.

3. Vorrichtung nach Anspruch 1 oder 2, **dadurch gekennzeichnet, dass** die Mittel (17) zum Ansteuern der Blutpumpe (6) und des Absperrorgans (18) derart ausgebildet sind, dass nach dem Anhalten der Blutpumpe (6) das Absperrorgan (18) mehrfach zumindest teilweise geschlossen und zumindest teilweise geöffnet wird.

4. Vorrichtung nach einem der Ansprüche 1 bis 3, **dadurch gekennzeichnet, dass** das Absperrorgan eine an der Blutzuführleitung (5) angeordnete Schlauchklemme (18) ist.

5. Vorrichtung nach einem der Ansprüche 1 bis 4, **dadurch gekennzeichnet, dass** die Mittel (25A) zum Senden von elektromagnetischer Strahlung als Mittel zum Senden von elektromagnetischer Strahlung mit einer ersten Wellenlänge und einer zweiten Wellenlänge ausgebildet sind, die sich voneinander unterscheiden.

6. Vorrichtung nach einem der Ansprüche 1 bis 5, **dadurch gekennzeichnet, dass** die Mittel (25A) zum Senden von elektromagnetischer Strahlung als Mittel zum Senden von elektromagnetischer Strahlung aus unterschiedlichen Richtungen ausgebildet sind, die orthogonal zueinander sind.

7. Vorrichtung nach einem der Ansprüche 1 bis 6, **dadurch gekennzeichnet, dass** die Mittel (25B) zum Empfangen von elektromagnetischer Strahlung als Mittel zum Empfangen von elektromagnetischer Strahlung aus unterschiedlichen Richtungen ausgebildet sind, die orthogonal zueinander sind.

8. Vorrichtung nach einem der Ansprüche 1 bis 7, **dadurch gekennzeichnet, dass** die elektromagnetische Strahlung Licht mit einer Wellenlänge zwischen 385 nm und 950 nm ist.

9. Vorrichtung nach einem der Ansprüche 1 bis 8, **dadurch gekennzeichnet, dass** der Blutbestandteil Glukose ist.

10. Vorrichtung zur extrakorporalen Blutbehandlung, die einen durch eine semipermeable Membran (2) in eine erste Kammer (3) und eine zweite Kammer (4) unterteilten Dialysator (1) oder Filter und ein Schlauchleitungssystem (I, II) umfasst, das für elektromagnetische Strahlung durchlässige Schlauchleitungen (5, 7; 10, 11) aufweist, mit einer Vorrichtung zur Messung eines Blutbestandteils nach einem der Ansprüche 1 bis 9.

**Claims**

1. Apparatus for measuring a blood component in the blood, for an extracorporeal blood treatment apparatus that comprises a dialyzer or filter divided into a first chamber and a second chamber by means of a semi-permeable membrane, and a tubing system that has a blood feed line (5) that leads to the first chamber of the dialyzer (1) or filter, and a blood return line (7) that departs from the first chamber of the dialyzer (1) or filter, and that has tubing that is essentially permeable for electromagnetic radiation,
   wherein the apparatus for measuring a blood component has:

   a measurement arrangement (21) having means (24) for transmitting electromagnetic radiation that enters into one of the lines of the tubing system at a measurement location, and means (25) for receiving electromagnetic radiation that exits from the tubing at the measurement location, wherein the measurement arrangement delivers measurement data that are characteristic for the intensity of the electromagnetic radiation entering at the measurement location and exiting from the tubing at the measurement location,
   means (6, 18) for changing the flow behavior of the fluid that flows in the tubing at the measurement location, means (24) for analyzing the measurement data of the measurement arrangement (21) obtained during the change in the flow behavior, and for determining the concentration of the blood component from the measurement data,
   **characterized in that**
   the means (6, 18) for changing the flow behavior have a blood pump (6) disposed in the blood feed line (5) or blood return line (7), for conveying blood, a shut-off element (18) disposed in the blood feed line (5) or blood return line (7), and means (17) for controlling the blood pump and the shut-off element, which are configured in such a manner that the flow rate of the blood in the blood feed line or blood return line is changed, wherein the measurement arrangement (21) is disposed on the blood feed line or blood return line, respectively, and that the means (17) for controlling the blood pump (6) and the shut-off element (18) are configured in such a manner that the blood pump (6) is stopped for a predetermined time interval, and after the blood pump is stopped, the shut-off element (18) is closed at least in part, particularly completely, and then opened again.

2. Apparatus according to claim 1, **characterized in that** the means (6, 18) for changing the flow behavior of the blood flowing in the blood feed line or blood return line have a blood pump disposed in the blood feed line (5), for conveying blood, and a shut-off element (18) disposed in the blood feed line (5).

3. Apparatus according to claim 1 or 2, **characterized in that** the means (17) for controlling the blood pump (6) and the shut-off element (18) are configured in such a manner that after the blood pump (6) is stopped, the shut-off element (18) is closed at least in part and opened at least in part, multiple times.

4. Apparatus according to one of claims 1 to 3, **characterized in that** the shut-off element is a tube clamp (18) disposed on the blood feed line.

5. Apparatus according to one of claims 1 to 4, **characterized in that** the means (25A) for transmitting electromagnetic radiation are configured as means for transmitting electromagnetic radiation having a first wavelength and a second wavelength, which are different from one another.

6. Apparatus according to one of claims 1 to 5, **characterized in that** the means (25A) for transmitting electromagnetic radiation are configured as means for transmitting electromagnetic radiation from different directions, which are orthogonal to one another.

7. Apparatus according to one of claims 1 to 6, **characterized in that** the means (25B) for receiving electromagnetic radiation are configured as means for receiving electromagnetic radiation from different directions, which are orthogonal to one another.

8. Apparatus according to one of claims 1 to 7, **characterized in that** the electromagnetic radiation is light at a wavelength between 385 nm and 950 nm.

9. Apparatus according to one of claims 1 to 8, **characterized in that** the blood component is glucose.

10. Apparatus for extracorporeal blood treatment apparatus, which comprises a dialyzer (1) or filter divided into a first chamber (3) and a second chamber (4) by means of a semi-permeable membrane (2) and has tubing (5, 7; 10, 11) that is permeable for electromagnetic radiation, having an apparatus according to one of claims 1 to 9.

**Revendications**

1. Dispositif de mesure d'un composant sanguin dans le sang pour un dispositif de traitement extracorporel du sang, qui comprend un dialyseur ou un filtre subdivisé par une membrane semi-perméable en une première chambre et une deuxième chambre et un système de conduite flexible qui comporte une conduite d'acheminement du sang (5) conduisant à la première chambre (3) du dialyseur (1) ou du filtre et une conduite de retour du sang (7) partant de la première chambre du dialyseur ou du filtre et qui comporte des conduites flexibles essentiellement perméables au rayonnement électromagnétique,
dans lequel le dispositif de mesure d'un composant sanguin présente :

un agencement de mesure (21) doté de moyens (24) pour envoyer un rayonnement électromagnétique qui entre dans l'une des conduites flexibles du système de conduites flexibles sur un point de mesure, et des moyens (25) pour recevoir un rayonnement électromagnétique qui sort sur le point de mesure de la conduite flexible, l'agencement de mesure fournissant des données de mesure caractéristiques pour l'intensité du rayonnement électromagnétique entrant dans la conduite flexible sur le point de mesure et sortant de la conduite flexible sur le point de mesure,
des moyens (6, 18) pour modifier le comportement d'écoulement d'un liquide s'écoulant sur le point de mesure dans la conduite flexible,
des moyens (24) pour analyser les données de mesure obtenues pendant la modification du comportement d'écoulement de l'agencement de mesure (21) et pour déterminer la concentration du composant sanguin à partir des données de mesure,
**caractérisé en ce que**
les moyens (6, 18) pour modifier le comportement d'écoulement présentent une pompe de sanguine (6) disposée dans la conduite d'acheminement du sang (5) ou dans la conduite de retour du sang (7) pour transporter le sang, un organe de blocage (18) disposé dans la conduite d'acheminement du sang (5) ou dans la conduite de retour du sang (7) et des moyens (17) pour commander la pompe de sanguine et l'organe de blocage qui sont conçus de telle sorte que le débit de sang dans la conduite d'acheminement du sang ou dans la conduite de retour du sang soit modifié, l'agencement de mesure (21) étant disposé sur la conduite d'acheminement du sang ou la conduite de retour du sang, et
**en ce que** les moyens (17) pour commander la pompe de sanguine (6) et l'organe de blocage (18) sont conçus de telle sorte que la pompe de sanguine (6) soit arrêtée pendant un intervalle de temps prédéterminé et qu'après l'arrêt de la pompe de sanguine, l'organe de blocage (18) soit fermé au moins partiellement, en particulier complètement, puis soit à nouveau ouvert.

2. Dispositif selon la revendication 1, **caractérisé en ce que** les moyens (6, 18) pour modifier le comportement d'écoulement du sang s'écoulant dans la conduite d'acheminement de sang ou la conduite de retour du sang présentent une pompe de sanguine (6) disposée dans la conduite d'acheminement du sang (5) pour transporter le sang et un organe de blocage (18) disposé dans la conduite d'acheminement du sang (5).

3. Dispositif selon la revendication 1 ou 2, **caractérisé en ce que** les moyens (17) pour commander la pompe de sanguine (6) et l'organe de blocage (18) sont conçus de telle sorte qu'après l'arrêt de la pompe de sanguine (6), l'organe de blocage (18) est plusieurs fois au moins partiellement fermé et au moins partiellement ouvert.

4.  Dispositif selon l'une des revendications 1 à 3, **caractérisé en ce que** l'organe de blocage est un collier de serrage (18) disposé sur la conduite d'acheminement du sang (5).

5.  Dispositif selon l'une des revendications 1 à 4, **caractérisé en ce que** les moyens (25A) pour émettre un rayonnement électromagnétique sont conçus comme des moyens pour émettre un rayonnement électromagnétique avec une première longueur d'ondes et une deuxième longueur d'ondes qui se distinguent l'une de l'autre.

6.  Dispositif selon l'une des revendications 1 à 5, **caractérisé en ce que** les moyens (25A) pour émettre un rayonnement électromagnétique sont conçus comme des moyens pour émettre un rayonnement électromagnétique à partir de directions différentes qui sont orthogonales les unes par rapport aux autres.

7.  Dispositif selon l'une des revendications 1 à 6, **caractérisé en ce que** les moyens (25B) pour recevoir un rayonnement électromagnétique sont conçus comme des moyens pour recevoir un rayonnement électromagnétique à partir de directions différentes qui sont orthogonales les unes par rapport aux autres.

8.  Dispositif selon l'une des revendications 1 à 7, **caractérisé en ce que** le rayonnement électromagnétique est une lumière ayant une longueur d'ondes entre 385 nm et 950 nm.

9.  Dispositif selon l'une des revendications 1 à 8, **caractérisé en ce que** le composant sanguin est le glucose.

10. Dispositif pour le traitement extracorporel du sang, qui comprend un dialyseur (1) ou un filtre subdivisé par une membrane semi-perméable (2) en une première chambre (3) et une deuxième chambre (4) et un système de conduite flexible (I, II) qui présente des conduites flexibles (5, 7 ; 10, 11) perméables au rayonnement électromagnétique, avec un dispositif de mesure d'un composant sanguin selon l'une des revendications 1 à 9.

Fig. 1

EP 2 416 699 B1

**Fig. 2**

**Fig. 3**

**Fig. 4**

Fig. 5

Fig. 6

Fig. 7

Fig. 8

Fig. 9

**IN DER BESCHREIBUNG AUFGEFÜHRTE DOKUMENTE**

**In der Beschreibung aufgeführte Patentdokumente**

- WO 2006006153 A1
- WO 2007020647 A1
- WO 2004105596 A1
- EP 1083948 B1

- US 2004129616 A1
- US 2007083145 A1
- US 5312550 A
- WO 2004057313 A1

**In der Beschreibung aufgeführte Nicht-Patentliteratur**

- **ILYA FINE et al.** Occlusion Spectroscopy as a New Paradigm for Non-Invasive Blood Measurement. *Proceedings of SPIE,* 2001, vol. 4263, 122-130